# EUROPEAN PATENT SPECIFICATION

(11) **EP 3 482 793 B1**
(45) Date of publication and mention of the grant of the patent: **16.09.2020**
(21) Application number: 18000893.0
(22) Date of filing: 13.11.2018
(51) Int. Cl.: A61M 37/00

(54) **TATTOO APPARATUS**
TÄTOWIERUNGSGERÄT
APPAREIL POUR TATOUAGES

(30) Priority: 13.11.2017 IT 201700129372
(43) Date of publication of application: 15.05.2019
(73) Proprietor: d'Amico, Rosario, 10134 Torino (TO) (IT); Lucarelli, Monica, 10134 Torino (TO) (IT)
(72) Inventor: D'Amico, Rosario, I-10134 Torino (TO) (IT)
(74) Representative: Aprà, Mario

(56) References cited:
- WO-A1-2015/094042
- WO-A1-2015/156715
- CA-A1- 2 923 061
- CN-U- 206 492 106
- US-A1- 2009 125 049

## Description

The present invention relates to a tattoo apparatus.

A tattoo apparatus comprises a pen, gripped and used by the operator to draw tattoos by introducing pigments into the dermis.

A known type of tattoo apparatus comprises an electromagnetic coil system that, appropriately supplied with electrical current, cause a linear movement of a metal bar, contained in the pen. At a free end of the bar there are one or more needles that penetrate the skin and leave pigments in the dermis. The speed with which the needles penetrate the skin is, for example, around 50 penetrations per second.

Another known type of tattoo apparatus instead comprises an electric motor with variable power, that allows the needles to penetrate the dermis. This other type of apparatus also comprises a needle bar contained in the pen and operating as described above.

Both known tattoo apparatuses comprise an electrical current power supply with adjustable voltage current and provided with a safety fuse. This power supply is connected with respect to a power supply network by means of a cable.

The tattoo apparatus, both of the type with electromagnetic coils and of the type with electric motor, is provided with an electrical connection, by means of a flexible electrical cable, with respect to an electric current outlet of the power supply, which is produced as a separate and stationary unit. In particular, the electromagnetic coil system or the electric motor of the apparatus is provided with electrical connection means with a flexible cable with respect to said power supply. Moreover, the known tattoo apparatus comprises a pedal device to control the electrical power supply to the power supply unit and connected to the network by means of an electrical cable. The pedal control device is used to activate / deactivate the apparatus and to adjust, during the operation thereof, delivery of the electrical power that moves the needle bar. Therefore, during operation of the apparatus, the operator must always maintain the pedal control device pressed with one foot.

It will be noted that, by means of the aforesaid arrangement of parts, the movements of the operator gripping the pen to draw a tattoo are restricted, on the one hand by the flexible power supply cable connected to the power supply and, on the other hand, by the pedal control device, which the operator must operate continuously by means of one food to control and adjust the electrical power supply to this pen. This double spatial restriction considerably reduces the operator's freedom of movement and hence the possibility of drawing the tattoo precisely and according to more or less complex designs. In particular, only operators with considerable expertise are able to easily and naturally coordinate all the movements required to draw the tattoo, in the presence of the above-mentioned restrictions of space and movement.

Moreover, when the operator requires to change the electrical parameters to draw different types of tattoo it is necessary to disconnect the electrical power supply in order to reset the parameters.

On the other hand, there is known a tattoo apparatus, configured as single portable unit, which is operatively worn on a forearm of the operator and comprises a supporting wrist band connected to this forearm, as well as electric circuit means, integral with said wrist band and including electrical power supply battery means, on/off electrical switch means for connection / disconnection of said electric circuit means and potentiometer electric means with respective manual adjustment means of the electrical power delivered by said electric circuit means, to a tattoo pen, which is detachably electrically connected with respect to said potentiometer means. It can be noted that, for functional needs, the known apparatus is connected to the forearm of the hand that grips the pen. Moreover, during operation, the operator needs to be able to directly see the aforesaid manual means for adjusting the electrical power delivered, in order to be able to vary the power according to need, with the hand of the other forearm. Therefore, operating arrangement of said known tattoo apparatus on the aforesaid forearm is obligatory. However, this known tattoo apparatus, configured as single portable unit, is relatively heavy, which has a significant impact on the forearm and related hand that carries out the movements required to draw the tattoo, thereby limiting, both due to fatigue and to overall dimensions, operating ability and freedom of movement. The less experienced the operator is, the more significant this limit will be.

US 2009/125049 A1 discloses a tattoo apparatus comprising a foot-operated speed controller and a hand-held tattoo machine both connected via cables to a power supply.

The present invention intends to provide a solution to the aforesaid problems. An object of the present invention is to provide a tattoo apparatus, that allows the operator full freedom of movement, specifically of the arm and of the hand that draws the tattoo on the body of a person. In particular, the object of the present invention is to facilitate even an inexperienced operator in the operations to draw a tattoo.

Another object of the present invention is to provide a tattoo apparatus, which is ergonomic and allows the weight that burdens the hand of the operator performing the operations to tattoo the body of a person to be greatly limited, allowing the operator to perform these operations in an easy and natural manner.

Yet another object of the invention is to provide a tattoo apparatus, which allows the power delivered during operation to be easily varied, even according to relatively complex preset operating patterns.

One more object of the invention is to provide a tattoo apparatus, which has a simplified structure, safe and reliable operation and a relatively limited cost.

In view of the aforesaid objects, the present invention provides a tattoo apparatus the essential characteristic of which forms the subject matter of claim 1. Further advantageous features of the invention are described in the dependent claims.

The aforesaid claims are intended as fully incorporated herein.

Other characteristics and advantages of the invention will be apparent from the following detailed description of an example of embodiment with reference to the drawing, which shows important details for the invention, and from the claims. The characteristics illustrated are not necessarily to scale and are represented so that the specific features according to the invention are clearly highlighted. The different characteristics can be produced individually or in any combination with one another, as variants of the invention.

In the accompanying drawing:
- Fig. 1 is a perspective and schematic view of the tattoo apparatus according to an example of embodiment of the invention, worn in the operating configuration by an operator, illustrated with dashed lines;
- Fig. 2 illustrates an elevation view of the tattoo apparatus according to Fig. 1 in detached state and with two different types of pen, alternative to each other, i.e., of the type with electromagnetic coil system and of the type with electric motor;
- Fig. 3 illustrates an elevation view of the tattoo apparatus according to Fig. 1 in the attached state, but not worn by an operator, and also shows a perspective view of a pedal switch device, optionally associated with the apparatus according to the invention;
- Fig. 4 is a partial view of the apparatus according to the invention, which illustrates a mobile telephone device with electrical connection means with respect to electric battery means of the tattoo apparatus according to the present example of embodiment of the invention.

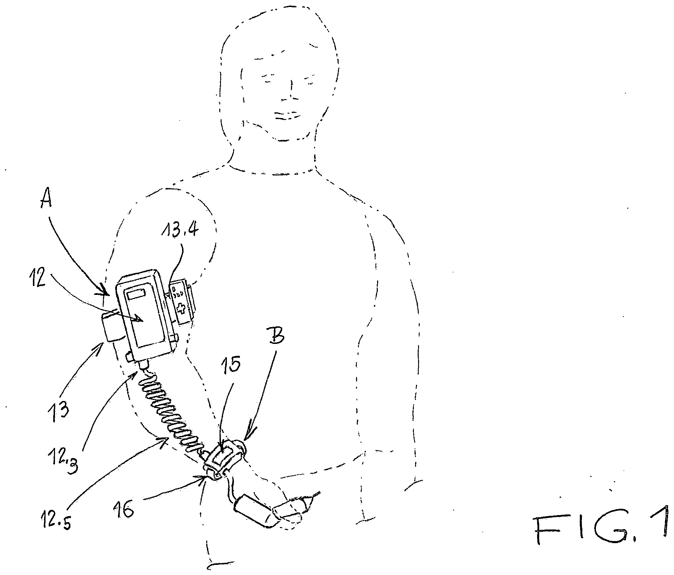

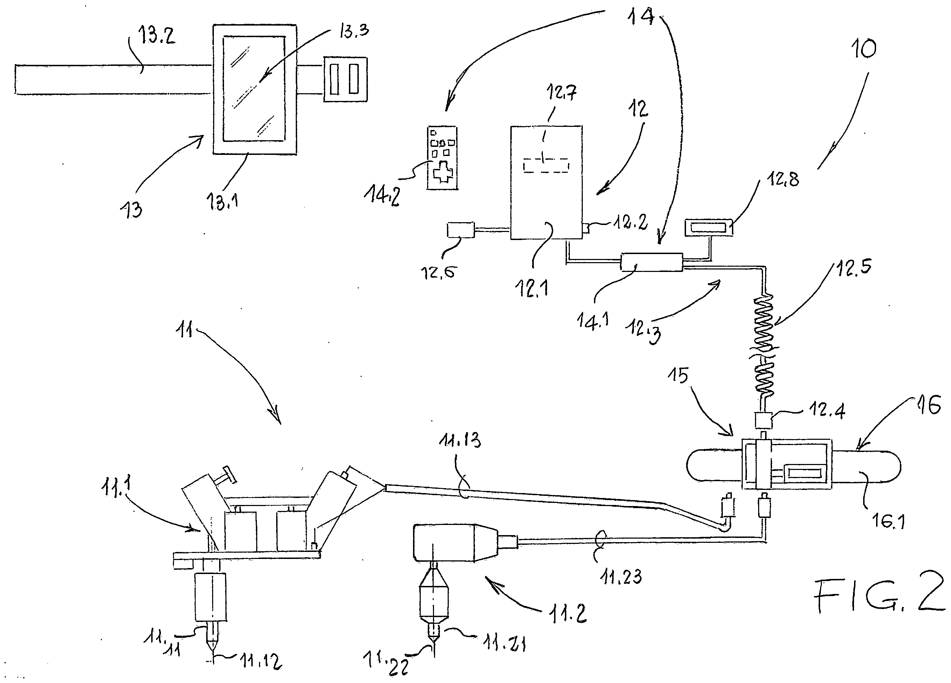

With reference to the accompanying drawing, the reference numeral 10 (Figs. 1 - 3) indicates as a whole the tattoo apparatus according to an example of embodiment of the present invention. Said tattoo apparatus 10 comprises a pen 11, gripped and used by the operator with one hand to draw tattoos by introducing pigments into the dermis of the person. Said pen 11, which is illustrated in Fig. 2 in two versions, alternative to each other, can include an electromagnetic coil system, indicated with 11.1, or electric motor means, indicated with 11.2. More in general, said pen 11 includes electrical control means (11.1, 11.2) that determine an alternating linear movement of a metal bar (11.11, 11.21 respectively), contained in this pen and that, at a free end, support one or more needles (11.12, 11.22 respectively), which by penetrating the skin of a person deposit pigments in the dermis of this person. Said pen 11 comprises flexible cable means and related electrical connector means (11.13, 11.23 respectively), for electrical connection with respect to a separate electrical supply and control circuit.

According to the invention, said tattoo apparatus 10 comprises, in addition to said pen 11, two distinct units for the electrical supply and control of said pen 11, which include respective electric circuit means, electrically isolated, and are worn separately by the operator on the same arm of the hand that grips said pen 11 to draw a tattoo, i.e., a first unit A (Fig. 1), detachably connected, by means of a first support means 13, with respect to the biceps of an arm of the operator and comprising first electric circuit means 12 to supply and control the electrical power delivered with respect to said pen 11, and a second unit B, detachably connected, by means of a second support means 16, with respect to the wrist of the arm of the operator and comprising second electric circuit means 15 for electrical connection between said first electric circuit means 12 and said pen 11, said first unit A and said second unit B being electrically connected to each other by means of flexible electrical cable connection means 12.3, 12.5, as illustrated by way of example in Fig. 1. Moreover, said pen 11 is detachably electrically connected with respect to said second electric circuit means 15 by means of said flexible cable means and related electrical connector (11.13, 11.23, respectively).

In particular, said tattoo apparatus 10 essentially comprises:
- first electric circuit means 12, which include electric battery means 12.1, electrically connected with respect to on/off electrical switch means 12.2, branched through which is a corresponding output electrical line 12.3, including electrical connector means 12.4 arranged at the end of a flexible electrical cable 12.5, and microcontroller electronic means (printed circuit, known *per se* and not illustrated), which are configured and programmed, in particular, to control the electrical power delivered by said electric battery means 12.1;
- remote control means 14 of said first electric circuit means 12 comprising:
   - electronic means 14.1 that receive encoded signals, for example encoded electromagnetic signals, electrically connected with respect to said first electric circuit means 12;
   - electronic means 14.2 that emit encoded signals, for example encoded electromagnetic signals, configured to send at least one encoded signal, which is received by said electronic means (14.1) that receive encoded signals;
   - second electric circuit means 15, including an electric circuit configured for the detachable electrical connection of said electrical connector means 12.4 of said first electric circuit means 12 and of said electrical connector means (11.13, 11.23 respectively) of said pen 11.

It can be noted that said microcontroller electronic means are electrically connected with respect to said electronic means 14.1 that receive encoded signals. In particular, said apparatus 10 according to the example illustrated comprises:
- said first support means 13 which can be worn with respect to the biceps of an arm of the operator and configured as support for said first unit A, comprising at least said electric battery means 12.1, said on/off electrical switch means 12.2 and said microcontroller electronic means, and
- said second support means 16 that can be worn with respect to the wrist of the same arm of the operator and configured as support for said second unit B and comprising at least said second electric circuit means 15.

Advantageously, said first wearable support means 13 is configured in the fashion of a pocket 13.1 and comprises fastenable strip means 13.2 for detachable connection with respect to the biceps of an arm of the operator, while said second wearable support means 16 is configured in the fashion of a rigid wrist band 16.1 for detachable connection with respect to the wrist of the same arm of the operator. In this way, as illustrated in Fig. 1, after having put on said first support means 13 and said second support means 16 and after having electrically and mechanically connected said electric line 12.3, 12.5 output from said first electric circuit means 12 with respect to said second electric circuit means 15, the operator electrically and mechanically connects the pen 11 with respect to said same second electric circuit means 15 and grips this pen to draw a tattoo with the corresponding hand. In this condition, the operator can move the arm and the corresponding hand that grips the pen 11 freely, while the weight of the apparatus 10 is distributed along said arm, in particular the heaviest part, i.e. the part containing the electric battery means 12.1, is connected to the biceps of the same arm, close to the shoulder.

Preferably, said battery means 12.1 are of the rechargeable electric battery type, for example comprising a lithium-ion polymer battery, for example 12V with 3000mAh, 4800mAh and 9800mAh, or 24V with 3000mAh.

Moreover, said first electric circuit means 12 comprise detachable electrical connection means 12.6 with respect to corresponding electrical connection means of an external power supply network, for recharging said battery means 12.1. Said electrical connection means 12.6 comprise, for example, an electrical current outlet including an electrical connector, for example of the type with USB electrical outlet.

Moreover, said first electric circuit means 12 comprise electronic display means 12.7 connected with respect to said electric battery means 12.1 and which are configured to display the level of electrical charge of the same electric battery means 12.1.

In the example illustrated, said first connection means 13 configured as a pocket 13.1 have a transparent wall 13.3 of the pocket 13.1, through which said electronic display means 12.7 are visible from outside this pocket.

On the other hand and by way of example, to control the electrical power delivered, said microcontroller electronic means carry out, when activated, current pulse width modulation, consisting of varying the direct current electrical power, acting on the duration of the supply of the charge of the electric battery means 12.1 according to an on/off method, known as change-over switching or, for switching of longer duration, known as intermittent switching.

According to the example illustrated, said electronic means 14.1 that receive encoded signals are electrically connected with respect to the output electric line 12.3 of said first electric circuit means 12, with respect to which further electronic display means 12.8 are also connected, for example configured to display the level of charge of said electric battery means 12.1 or other electrical quantities, such as the electrical power delivered by said first electric circuit means 12 to said pen 11.

Said electronic means 14.2 that emit encoded signals, for example electromagnetic, are preferably configured to selectively send an encoded signal of a plurality of encoded signals, which is received by said electronic means 14.1 that receive encoded signals, for example electromagnetic. Said encoded signals identify, selectively and respectively, by way of example, the on/off status of said first electric circuit means 12; different levels of electrical power delivered by said first electric circuit means 12; the condition of constant or intermittent supply of electrical current to the pen 11, with consequent variations in frequency or duration of the electrical signal.

It can be noted that said electronic means 14.2 that emit encoded signals are configured as push-button remote control device and said first support means 13 is provided with connection means 13.4 (Fig. 1) of said remote control device 14.2.

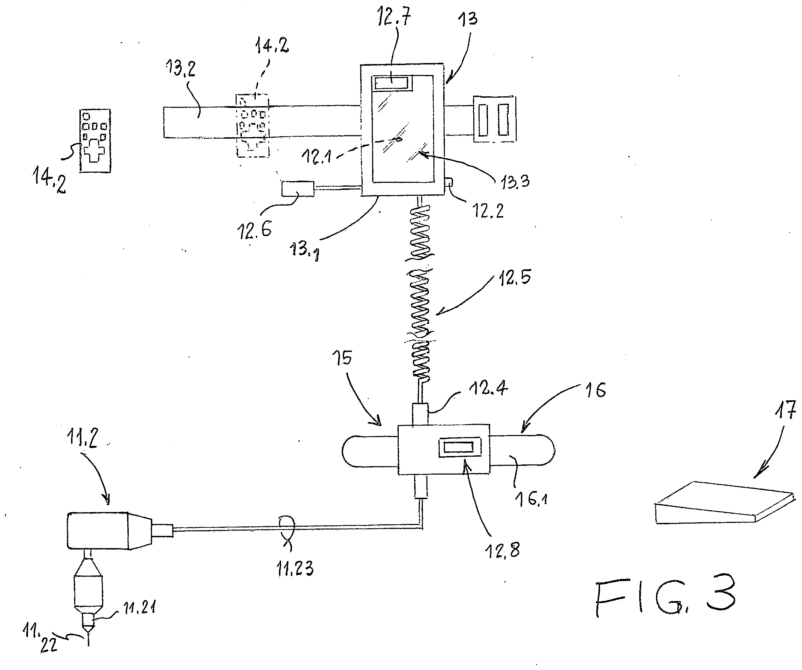

According to a variant of embodiment of the invention, said apparatus 10 further comprises an on/off electrical pedal switch means 17 (Fig. 3) that comprises electronic means that emit encoded signals, for example electromagnetic, configured to send at least one encoded signal, which is received by said electronic means 14.1 that receive encoded signals, wherein said encoded signals received by said receiving electronic means 14.1 determine, selectively and respectively, the state of electrical connection / disconnection of said electric battery means 12.1 of said first electric circuit means 12.

It can be noted that, according to a variant of embodiment of the tattoo apparatus according to the invention, said second unit (B) includes said second electric circuit means 15, comprising an electric circuit configured for detachable electrical and mechanical connection with respect to said electrical connector means 12.4 of said first electric circuit means 12 and said electrical connector means (11.13, 11.23) of said pen 11, and microcontroller electronic means, configured and programmed to control the electrical power delivered by said electric battery means 12.1 of said first electric circuit means 12.

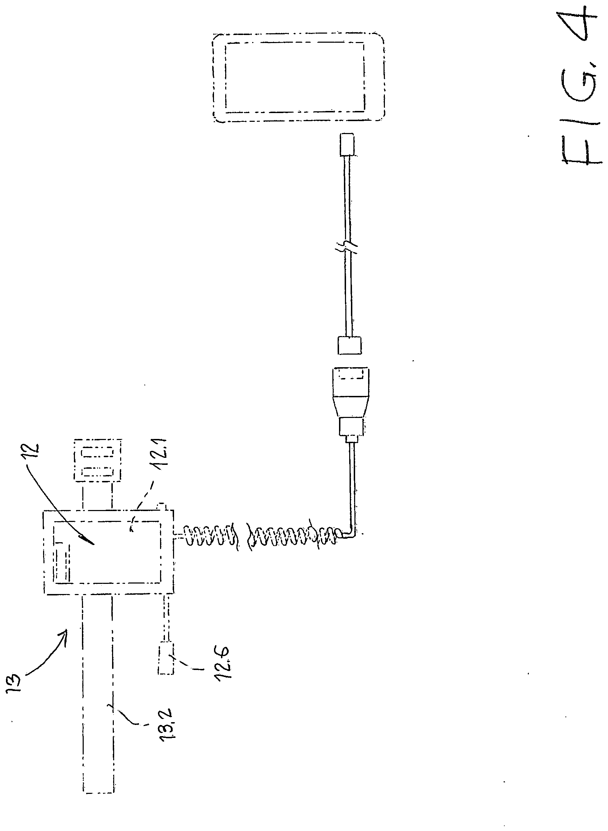

Fig. 4 of the drawing illustrates the connection between the electric battery means (not illustrated) of a battery-operated electronic device, such as a mobile telephone, and the electric battery means 12.1 of said first electric circuit means 12 housed in the first support means 13 of the apparatus 10, by means of a suitable flexible cable with electrical connection means, alternative to the electrical connection means of the flexible cable 12.5.

## Claims

1. Tattoo apparatus (10), comprising a pen (11), gripped and used by the operator with one hand to draw tattoos, including electrical control means (11.1, 11.2) that determine an alternating movement of a bar (11.11, 11.21), which supports one or more needles (11.12, 11.22), which by penetrating the skin of a person deposit pigments in the dermis of this person, said pen (11) comprising flexible cable means and related electrical connector means (11.13, 11.23), for the detachable electrical connection with respect to a separate electrical supply and control circuit, **characterized in that** it comprises two distinct units (A, B) for the electrical supply and control of said pen (11), which include respective electric circuit means (12, 15), electrically isolated, and which are worn separately on the arm of the operator corresponding to the hand that grips said pen (11), i.e., a first unit (A), detachably connected, by means of a first support means (13), with respect to the biceps of said arm of the operator and comprising first electric circuit means (12), and a second unit (B), detachably connected, by means of a second support means (16), with respect to the wrist of the same arm of the operator and comprising second electric circuit means (15), wherein said first electric circuit means (12) and said second electric circuit means (15) are electrically connected to each other by means of flexible electrical cable connection means (12.3, 12.5) and said pen (11) is detachably electrically connected with respect to said second electric circuit means (15).

2. Tattoo apparatus (10) according to claim 1, **characterized in that** it comprises:
- said first electric circuit means (12), which include electric battery means (12.1), electrically connected with respect to on/off electrical switch means (12.2), branched through which is a corresponding output electrical line (12.3), including electrical connector means (12.4) arranged at the end of a flexible electrical cable (12.5);
- remote control means (14) of said first electric circuit means (12) comprising:
- electronic means (14.1) that receive encoded signals, electrically connected with respect to said first electric circuit means (12);
- electronic means (14.2) that emit encoded signals, configured to send at least one encoded signal to said electronic means (14.1) that receive encoded signals;
- said second electric circuit-means (15), including an electric circuit configured for the detachable electrical connection of said electrical connector means (12.4) of said first electric circuit means (12) and of said electrical connector means (11.13, 11.23) of said pen (11).

3. Tattoo apparatus (10) according to claims 1 and 2, **characterized in that** said first unit (A) includes said first electric circuit means (12) comprising said electric battery means (12.1), said on/off electrical switch means (12.2) electrically branched with respect to said electric battery means (12.1), microcontroller electronic means, configured and programmed to control the electrical power delivered by said electric battery means (12.1) and electrically connected with respect to said electronic means (14.1) that receive encoded signals.

4. Tattoo apparatus (10) according to claim 2, **characterized in that** said second unit (B) includes said second electric circuit means (15), comprising an electric circuit configured for detachable electrical and mechanical connection with respect to said electrical connector means (12.4) of said first electric circuit means (12) and said electrical connector means (11.13, 11.23) of said pen (11), microcontroller electronic means, configured and programmed to control the electrical power delivered by said electric battery means (12.1) of said first electric circuit means (12).

5. Tattoo apparatus (10) according to one or more of the preceding claims, **characterized in that** said first wearable support means (13) is configured in the fashion of a pocket (13.1) and comprises fastenable strip means (13.2) for detachable connection with respect to the biceps of an arm of the operator, and said second wearable support means (16) is configured in the fashion of a wrist band (16.1) for detachable connection with respect to the wrist of the same arm of the operator.

6. Tattoo apparatus (10) according to one or more of the preceding claims, **characterized in that** said first electric circuit means (12) comprise detachable electrical connection means (12.6) with respect to corresponding electrical connection means of an external power supply network, for recharging said battery means (12.1).

7. Tattoo apparatus (10) according to one or more of the preceding claims, **characterized in that** said first electric circuit means (12) comprise electronic display means (12.7) electrically connected with respect to said electric battery means (12.1) and configured to display the level of electrical charge of the electric battery means (12.1).

8. Tattoo apparatus (10) according to one or more of the preceding claims, **characterized in that** it comprises electronic display means (12.8) electrically connected with respect to said first electric circuit means (12) and configured to display the electrical power delivered by said first electric circuit means (12) to said pen (11).

9. Tattoo apparatus (10) according to one or more of the preceding claims, **characterized in that** said electronic means (14.2) that emit encoded signals are configured to selectively send one encoded signal of a plurality of encoded signals, which is received by said electronic means (14.1) that receive encoded signals, and **in that** said encoded signals received identify, selectively and respectively, the on or off state of said first electric circuit means (12), a plurality of different levels of electrical power delivered by said first electric circuit means (12) to said pen (11), a plurality of modes of supplying the electrical current delivered through said first electric circuit means (12) to said pen (11).

10. Tattoo apparatus (10) according to one or more of the preceding claims, **characterized in that** said electronic means (14.2) that emit encoded signals are configured as push-button remote control device and said first support means (13) is provided with detachable connection means (13.4) of said remote control device (14.2).

11. Tattoo apparatus (10) according to one or more of claims 2 to 10, **characterized in that** it comprises an on/off electrical pedal switch means (17), comprising electronic means that emit encoded signals, configured to send at least one encoded signal to said electronic means (14.1) that receive encoded signals, wherein said encoded signals received by said receiving electronic means (14.1) determine, selectively and respectively, the state of electrical connection / disconnection of said electric battery means (12.1) of said first electric circuit means (12).

## Patentansprüche

1. Tätowiervorrichtung (10), umfassend einen Stift (11), der vom Bediener mit einer Hand ergriffen und zum Zeichnen von Tätowierungen verwendet wird, einschließlich elektrischer Steuermittel (11.1, 11.2), die eine abwechselnde Bewegung einer Stange (11.11, 11.21) bestimmen, die trägt eine oder mehrere Nadeln (11.12, 11.22), die durch Eindringen in die Haut einer Person Pigmente in der Dermis dieser Person ablagern, wobei der Stift (11) flexible Kabelmittel und zugehörige elektrische Verbindungsmittel (11.13, 11.23) umfasst für die abnehmbare elektrische Verbindung in Bezug auf eine separate elektrische Versorgungs- und Steuerschaltung, **dadurch gekennzeichnet, dass** sie zwei separate Einheiten (A, B) für die elektrische Leistung und Steuerung des Stifts (11) umfasst, die entsprechende elektrische Schaltungsmittel (12, 15) enthalten, die elektrisch isoliert sind und die separat am Arm des Bedieners getragen werden, entsprechend der Hand, die den Stift (11) hält, d.h. eine erste ein Einheit (A), die auf abnehmbare Weise mittels einer ersten Stützeinrichtung (13) in Bezug auf den Bizeps des Bedienerarms verbunden ist und die erste elektrische Schaltungseinrichtung (12) und eine zweite Einheit (B) umfasst, die verbunden sind zerlegbar mittels eines zweiten Stützmittels (16) in Bezug auf das Handgelenk desselben Arms des Bedieners und umfassend ein zweites elektrisches Schaltungsmittel (15), wobei das erste elektrische Schaltungsmittel (12) und das zweite Mittel des Stromkreises (15) sind mittels Verbindungsmitteln mit flexiblem Elektrokabel (12.3, 12.5) elektrisch miteinander verbunden, und der Stift (11) ist in Bezug auf das zweite Stromkreismittel (15) auf abnehmbare Weise elektrisch verbunden.

2. Tätowiervorrichtung (10) nach Anspruch 1, **dadurch gekennzeichnet, dass** sie umfasst:
die erste elektrische Schaltungseinrichtung (12), die elektrische Batteriemittel (12.1) umfasst, die in Bezug auf die elektrischen Ein / Aus-Schaltmittel (12.2) elektrisch verbunden sind, durch die eine entsprechende elektrische Ausgangsleitung (12.3) verzweigt ist, einschließlich der elektrischen Verbindungsmittel (12.4), die am Ende eines flexiblen elektrischen Kabels (12.5) angeordnet sind;
- Fernsteuermittel (14) der ersten elektrischen Schaltungseinrichtung (12), umfassend:
- elektronische Mittel (14.1), die codierten Signale empfangen, die in Bezug auf die erste elektrische Schaltungseinrichtung (12) elektrisch verbunden sind;
- elektronische Mittel (14.2), die codierten Signale aussenden, die konfiguriert sind, um mindestens ein codiertes Signal an die elektronischen Mittel (14.1) zu senden, die codierten Signale empfangen;
- die zweite elektrische Schaltungseinrichtung (15), einschließlich einer elektrischen Schaltung, die für die abnehmbare elektrische Verbindung der elektrischen Verbindereinrichtung (12.4) der ersten elektrischen Schaltungseinrichtung (12) und der elektrischen Verbindereinrichtung (11.13, 11.23) von konfiguriert ist sagte Stift (11).

3. Tätowiervorrichtung (10) nach den Ansprüchen 1 und 2, **dadurch gekennzeichnet, dass** die erste Einheit (A) die erste elektrische Schaltungseinrichtung (12) umfasst, die die elektrische Batteriemittel (12.1) umfasst, wobei die elektrische Ein / Aus-Schalteinrichtung (12.2) elektrisch ist verzweigt in Bezug auf die elektrischen Batteriemittel (12.1), elektronische Mikrocontrollermittel, konfiguriert und programmiert, um die von den elektrischen Batteriemitteln (12.1) gelieferte elektrische Leistung zu steuern, und elektrisch verbunden in Bezug auf die elektronischen Mittel (14.1), die codierte Signale empfangen.

4. Tätowiervorrichtung (10) nach Anspruch 2, **dadurch gekennzeichnet, dass** die zweite Einheit (B) die zweite elektrische Schaltungseinrichtung (15) umfasst, die eine elektrische Schaltung umfasst, die für eine abnehmbare elektrische und mechanische Verbindung in Bezug auf die elektrische Verbindereinrichtung (12.4) konfiguriert ist. der ersten elektrischen Schaltungseinrichtung (12) und der elektrischen Verbindereinrichtung (11.13, 11.23) des Stiftes (11) eine elektronische Mikrocontrollereinrichtung, die konfiguriert und programmiert ist, um die von der elektrischen Batteriemittel (12.1) der ersten gelieferte elektrische Leistung zu steuern Stromkreismittel (12).

5. Tätowiervorrichtung (10) gemäß einem oder mehreren der vorhergehenden Ansprüche, **gekennzeichnet dadurch, dass** das erste tragbare Stützmittel (13) in der Art einer Tasche (13.1) konfiguriert ist und ein befestigbares Streifenmittel (13.2) zur abnehmbaren Verbindung in Bezug auf dies umfasst an den Bizeps eines Arms des Bedieners, und das zweite tragbare Stützmittel (16) ist in der Art eines Armbandes (16.1) für eine abnehmbare Verbindung in Bezug auf das Handgelenk desselben Arms des Bedieners konfiguriert.

6. Tätowiervorrichtung (10) nach einem oder mehreren der vorhergehenden Ansprüche, **gekennzeichnet dadurch, dass** die erste elektrische Schaltungseinrichtung (12) abnehmbare elektrische Verbindungseinrichtung (12.6) in Bezug auf entsprechende elektrische Verbindungseinrichtung eines externen Stromversorgungsnetzes zum Aufladen umfasst die Batterie bedeutet (12.1).

7. Tätowiervorrichtung (10) gemäß einem oder mehreren der vorhergehenden Ansprüche, **gekennzeichnet dadurch, dass** die erste elektrische Schaltungseinrichtung (12) elektronische Anzeigemittel (12.7) umfasst, die in Bezug auf die elektrischen Batteriemittel (12.1) elektrisch verbunden und konfiguriert sind, um die anzuzeigen Ladezustand der elektrischen Batteriemittel (12.1).

8. Tätowiervorrichtung (10) nach einem oder mehreren der vorhergehenden Ansprüche, **gekennzeichnet dadurch, dass** sie elektronische Anzeigemittel (12.8) umfasst, die in Bezug auf das erste elektrische Schaltungsmittel (12) elektrisch verbunden sind und konfiguriert sind, um die von dem ersten gelieferte elektrische Leistung anzuzeigen Stromkreismittel (12) zu dem Stift (11).

9. Tätowiervorrichtung (10) gemäß einem oder mehreren der vorhergehenden Ansprüche, **gekennzeichnet dadurch, dass** die elektronischen Mittel (14.2), die codierte Signale aussenden, so konfiguriert sind, dass sie selektiv ein codiertes Signal einer Vielzahl von codierten Signalen senden, das von den elektronischen Mitteln empfangen wird (14.1) die codierte Signale empfangen und in denen die empfangenen codierten Signale selektiv bzw. den Ein- oder Ausschaltzustand der ersten elektrischen Schaltungseinrichtung (12) mehrere unterschiedliche Niveaus elektrischer Leistung identifizieren, die von der ersten elektrischen Schaltung geliefert werden Mittel (12) zu dem Stift (11), eine Vielzahl von Modi zum Zuführen des elektrischen Stroms, der durch das erste elektrische Schaltungsmittel (12) zu dem Stift (11) geliefert wird.

10. Tätowiervorrichtung (10) gemäß einem oder mehreren der vorhergehenden Ansprüche, **gekennzeichnet dadurch, dass** die elektronischen Mittel (14.2), die codierte Signale aussenden, als Druckknopf-Fernbedienungsvorrichtung konfiguriert sind und die ersten Stützmittel (13) mit einer abnehmbaren Verbindung versehen sind bedeutet (13.4) der Fernbedienung (14.2).

11. Tätowiervorrichtung (10) nach einem oder mehreren der Ansprüche 2 bis 10, **gekennzeichnet dadurch, dass** sie eine elektrische Ein /Aus-Pedalschaltereinrichtung (17) umfasst, die elektronische Mittel umfasst, die codierte Signale aussenden, die konfiguriert sind, um mindestens ein codiertes Signal an zu senden die elektronischen Mittel (14.1), die codierte Signale empfangen, wobei die codierten Signale, die von den empfangenden elektronischen Mitteln (14.1) empfangen werden, selektiv bzw. den Zustand der elektrischen Verbindung / Trennung der elektrischen Batteriemittel (12.1) der ersten elektrischen Schaltung bestimmen bedeutet (12).

## Revendications

1. Appareil de tatouage (10), comprenant un stylo (11), saisi et utilisé par l'opérateur d'une main pour dessiner des tatouages, comprenant des moyens électriques de commande (11.1, 11.2) qui déterminent un mouvement alternatif d'une barre (11.11, 11.21), qui supporte une ou plusieurs aiguilles (11.12, 11.22) qui, en pénétrant dans la peau d'une personne, déposent des pigments dans le derme de cette personne, ledit stylo (11) comprenant un moyen à câble flexible et un moyen à connecteur électrique associé (11.13, 11.23), pour le connexion électrique détachable par rapport à un circuit électrique d'alimentation et de commande séparé, **caractérisée en ce qu'**il comprend deux unités distinctes (A, B) pour l'alimentation et la commande électriques dudit stylo (11), qui comprennent des moyens de circuit électrique respectifs (12, 15), isolés électriquement, et qui sont portés séparément sur le bras de l'opérateur correspondant à la main qui saisit ledit stylo (11), c'est-à-dire une première unité (A), connectée de manière amovible, au moyen d'un premier moyen de support (13), par rapport aux biceps dudit bras de l'opérateur et comprenant des premiers moyens de circuit électrique (12), et une deuxième unité (B), connectée de manière amovible, au moyen d'un deuxième moyen de support (16), par rapport au poignet du même bras de l'opérateur et comprenant des seconds moyens de circuit électrique (15), où lesdits premiers moyens de circuit électrique (12) et lesdits seconds moyens de circuit électrique (15) sont connectés électriquement l'un à l'autre par l'intermédiaire d'un moyen de connexion à câble électrique flexible (12.3, 12.5) et ledit stylo (11) est connecté électriquement de manière amovible par rapport auxdits seconds moyens de circuit électrique (15).

2. Appareil de tatouage (10) selon la revendication 1, **caractérisé en ce qu'**il comprend:
- lesdits premiers moyens de circuit électrique (12), qui comprennent des moyens à batterie électrique (12.1), connectés électriquement par rapport à des moyens de commutation électrique on/off (12.2), à travers lesquels est branchée une ligne électrique de sortie correspondante (12.3), comprenant des moyens à connecteur électrique (12.4) disposés à l'extrémité d'un câble électrique flexible (12.5);
- des moyens de commande à distance (14) desdits premiers moyens de circuit électrique (12) comprenant:
- des moyens électroniques (14.1) qui reçoivent des signaux codés, connectés électriquement par rapport auxdits premiers moyens de circuit électrique (12);
- des moyens électroniques (14.2) qui émettent des signaux codés, configurés pour envoyer au moins un signal codé auxdits moyens électroniques (14.1) qui reçoivent des signaux codés;
- lesdits seconds moyens de circuit électrique (15), comprenant un circuit électrique configuré pour la connexion électrique détachable desdits moyens à connecteur électrique (12.4) desdits premiers moyens de circuit électrique (12) et desdits moyens à connecteur électrique (11.13, 11.23) dudit stylo (11).

3. Appareil de tatouage (10) selon les revendications 1 et 2, **caractérisé en ce que** ladite première unité (A) comprend lesdits premiers moyens de circuit électrique (12) comprenant lesdits moyens à batterie électrique (12.1), lesdits moyens de commutation électrique on/off (12.2) électriquement branchés par rapport auxdits moyens à batterie électrique (12.1), des moyens électroniques à microcontrôleur, configurés et programmés pour contrôler la puissance électrique délivrée par lesdits moyen à batterie électrique (12.1) et connecté électriquement par rapport auxdits moyens électroniques (14.1) qui reçoivent des signaux codés.

4. Appareil de tatouage (10) selon la revendication 2, **caractérisé en ce que** ladite deuxième unité (B) comprend lesdits seconds moyens de circuit électrique (15), comprenant un circuit électrique configuré pour la connexion électrique et mécanique détachable par rapport auxdits moyens à connecteur électrique (12.4) desdits premiers moyens de circuit électrique (12) et lesdits moyens à connecteur électrique (11.13, 11.23) dudit stylo (11), des moyens électroniques à microcontrôleur, configurés et programmés pour contrôler la puissance électrique délivrée par lesdits moyens à batterie électrique (12.1) desdits premiers moyens de circuit électrique (12).

5. Appareil de tatouage (10) selon une ou plusieurs des revendications précédentes, **caractérisé en ce que** ledit premier moyen de support portable (13) est configuré à la manière d'une poche (13.1) et comprend un moyen à bande (13.2) pouvant être attaché pour une connexion détachable par rapport au biceps d'un bras de l'opérateur, et ledit second moyen de support portable (16) est configuré à la manière d'un bracelet (16.1) pour une connexion détachable par rapport au poignet du même bras de l'opérateur.

6. Appareil de tatouage (10) selon une ou plusieurs des revendications précédentes, **caractérisé en ce que** lesdits premiers moyens de circuit électrique (12) comprennent des moyens à connecteur électrique (12.6) détachables par rapport aux moyens à connecteur électrique correspondants d'un réseau d'alimentation externe, pour la recharge lesdits moyens à batterie (12.1).

7. Appareil de tatouage (10) selon une ou plusieurs des revendications précédentes, **caractérisé en ce que** lesdits premiers moyens de circuit électrique (12) comprennent des moyens d'affichage électronique (12.7) connectés électriquement par rapport auxdits moyens à batterie électrique (12.1) et configurés pour afficher le niveau de charge électrique des moyens à batterie électrique (12.1).

8. Appareil de tatouage (10) selon une ou plusieurs des revendications précédentes, **caractérisé en ce qu'**il comprend un moyen d'affichage électronique (12.8) connecté électriquement par rapport auxdits premiers moyens de circuit électrique (12) et configuré pour afficher la puissance électrique délivrée par lesdits premiers moyens de circuit électrique (12) audit stylo (11).

9. Appareil de tatouage (10) selon une ou plusieurs des revendications précédentes, **caractérisé en ce que** lesdits moyens électroniques (14.2) qui émettent des signaux codés sont configurés pour envoyer sélectivement un signal codé d'une pluralité de signaux codés, qui est reçu par lesdits moyens électroniques (14.1) qui reçoivent des signaux codés, et **en ce que** lesdits signaux codés reçus identifient, sélectivement et respectivement, l'état activé ou désactivé on/off desdits premiers moyens de circuit électrique (12), une pluralité de différents niveaux de puissance électrique délivrée par lesdits premiers moyens de circuit électrique (12) audit stylo (11), une pluralité de modes de fourniture du courant électrique délivré par lesdits premiers moyens de circuit électrique (12) audit stylo (11).

10. Appareil de tatouage (10) selon une ou plusieurs des revendications précédentes, **caractérisé en ce que** lesdits moyens électroniques (14.2) qui émettent des signaux codés sont configurés en tant que dispositif de télécommande à bouton-poussoir et ledit premier moyen de support (13) est muni de moyens connexion détachable (13.4) dudit dispositif de télécommande (14.2).

11. Appareil de tatouage (10) selon une ou plusieurs des revendications 2 à 10, **caractérisé en ce qu'**il comprend un interrupteur à pédale électrique on/off (17), comprenant des moyens électroniques qui émettent des signaux codés, configuré pour envoyer au moins un signal codé auxdits moyens électroniques (14.1) qui reçoivent des signaux codés, où lesdits signaux codés reçus par lesdits moyens électroniques de réception (14.1) déterminent, sélectivement et respectivement, l'état de connexion /déconnexion électrique desdits moyens à batterie électrique (12.1) desdits premiers moyens de circuit électrique (12).
